# EUROPEAN PATENT SPECIFICATION

(11) **EP 3 842 791 B1**
(45) Date of publication and mention of the grant of the patent: **04.10.2023**
(21) Application number: 19219306.8
(22) Date of filing: 23.12.2019
(51) Int. Cl.: G01N 21/84

(54) **ADJUSTMENT METHOD FOR ADJUSTING A SETUP FOR AN ANALYTICAL METHOD**
ANPASSUNGSVERFAHREN ZUM EINSTELLEN EINER EINRICHTUNG FÜR EIN ANALYTISCHES VERFAHREN
PROCÉDÉ DE RÉGLAGE POUR RÉGLER UNE CONFIGURATION POUR UN PROCÉDÉ ANALYTIQUE

(43) Date of publication of application: 30.06.2021
(73) Proprietor: Roche Diabetes Care GmbH, 68305 Mannheim (DE); F. Hoffmann-La Roche AG, 4070 Basel (CH)
(72) Inventor: BERG, Max, 68305 Mannheim (DE); KETH, Ingrid, 68305 Mannheim (DE); LIMBURG, Bernd, 68305 Mannheim (DE); SIEFFERT, Daniel, 68305 Mannheim (DE)
(74) Representative: Meinel, Anne Julia

(56) References cited:
- EP-A1- 3 581 921
- US-A1- 2013 330 831
- US-A1- 2015 233 898

## Description

### Technical Field

The present invention refers to an adjustment method for adjusting a setup for an analytical method of determining a concentration of an analyte in a bodily fluid based on a color formation reaction in an optical test. The invention further relates to a mobile device configured for performing the adjustment method, a computer program and a computer-readable storage medium for performing the adjustment method and a kit comprising a test strip or a dummy test strip and at least one of the mobile device, the computer program and the computer-readable storage medium. The method, device, computer program, storage media and kit specifically may be used in medical diagnostics, in order to for example qualitatively or quantitatively detect one or more analytes in one or more body fluids, such as for detecting glucose in blood and/or interstitial fluid. Other fields of application of the present invention, however, are feasible.

### Background art

In the field of medical diagnostics, in many cases, one or more analytes have to be detected in samples of a body fluid, such as blood, interstitial fluid, urine, saliva or other types of body fluids. Examples of analytes to be detected are glucose, triglycerides, lactate, cholesterol or other types of analytes typically present in these body fluids. According to the concentration and/or the presence of the analyte, an appropriate treatment may be chosen, if necessary. Without narrowing the scope, the invention specifically may be described with respect to blood glucose measurements. It shall be noted, however, that the present invention may also be used for other types of analytical measurements using test elements.

Generally, devices and methods known to the skilled person make use of test elements comprising one or more test chemicals, which, in presence of the analyte to be detected, are capable of performing one or more detectable detection reactions, such as optically detectable detection reactions. As an example, EP 0 821 234 A2 describes a diagnostic test carrier for the determination of an analyte from whole blood with the aid of a reagent system contained in the carrier and a method for the determination of an analyte from whole blood with the aid of the diagnostic test carrier. The diagnostic test carrier includes a color forming reagent. The test field has a sample application side to which the blood sample is delivered and a detection side where an optically detectable change occurs as a result of the reaction of the analyte with the reagent system. Furthermore, the test field is designed so that the erythrocytes contained in the sample do not reach the detection side. In addition, the test field comprises a transparent film and a first and a second superposed film layer applied thereto, wherein the first layer on the transparent film is substantially less light-scattering in the wet state than the overlying second layer.

With regard to the test chemicals comprised in test elements, reference may be made e.g. to J. Hoenes et al.: The Technology Behind Glucose Meters: Test Strips, Diabetes Technology & Therapeutics, Volume 10, Supplement 1, 2008, S-10 to S-26. Other types of test chemistry are possible and may be used for performing the present invention.

In analytical measurements, specifically analytical measurements based on color formation reactions, one technical challenge resides in the evaluation of the color change which is due to the detection reaction. Besides using dedicated analytical devices, such as handheld blood glucose meters, the use of generally available electronics such as smart phones and portable computers has become more and more popular over the recent years. WO 2012/131386 A1 discloses a testing apparatus for performing an assay, the testing apparatus comprising: a receptacle containing a reagent, the reagent being reactive to an applied test sample by developing a color or pattern variation; a portable device, e.g. a mobile phone or a laptop, comprising a processor and an image capture device, wherein the processor is configured to process data captured by the image capture device and output a test result for the applied test sample.

When using mobile computing devices such as smart phones, various influences need to be taken into account. Many product care activities like improving the measurement flow, improving the customer guidance, or optimizing user handling measurement performance are often based on user training sessions and provided feedback or observations, extensive market research, or complaints coming back from the market or training. Extensive market research or training, however, may be expensive and not continuously given. Further, it might be preferable to optimize user handling in advance, prior to receiving customers' complaints.

Various approaches have been developed over the recent years in order to improve user handling in the field of medical devices.

US 9,238,100 B2 discloses apparatuses and methods for training users of ambulatory medical devices. The methods relate to improving user interactions with the touchscreens of devices. In one embodiment there is an operating mode that records all user interactions along with various device parameters and allows the clinician to review the patient's performance for the initial use period. Automated analysis software may be employed to analyze the data generated by the device. The results of the analysis may be used by the clinician to improve the patient and device interaction.

US 2014/0205981 A1 discloses a dialysis treatment simulation system and method that comprise a treatment simulator, a computing module, and a user interface. The treatment simulator generates an event notification that simulates a notification of an event related to an interaction between a dialysis treatment machine and a patient. The computing module generates a request for a user action in response to the simulated event notification. The user interface outputs the simulated event notification and the request for the user action to an output device and provides a received user response to the computing module.

US 2015/194066 A1 relates to a smart peripheral device (defibrillator) for use in a medical training system. The medical training system having a processing device, software to emulate aspects of a medical device and a display in communication therewith. The processing device, software and display provide an interactive interface which emulates a portion of the medical device. The smart peripheral device includes a physical structure adapted to imitate a functional component of a medical device and at least one sensor adapted to measure an aspect of user performance, such as position, pressure or other physical variable.

EP 3138091 A1 discloses various systems and methods for injection training by collecting, processing, analyzing and displaying measured information associated with the delivery of an injection. Sensor-based measurements of a syringe's position and orientation in three-dimensional space are obtained and processed to provide metrics of a trainee's injection performance. The measurements can be combined with a digital model of a training apparatus to deliver a computer- generated, graphical depiction of the training injection, enabling visualization of the injection from perspectives unavailable in the physical world. The training injection execution, as reflected in the measured sensor-based data, can be reviewed and analyzed at times after, and in locations different than, the time and location of the training injection. Additionally, injection training data associated with multiple training injections can be aggregated and analyzed for, among other things, trends in performance.

US 2018/092595 A1 discloses systems and methods are provided for training and monitoring administration of an inhaler medication. The system includes a mobile computing device that is configured to provide an augmented reality training and monitoring aid for asthma patients. In particular, the mobile device is programmed to capture video using a camera and sound recordings using a microphone in order to measure the patient's head position from the video and measure events relating to inhalation and exhalation from the microphone recordings. This real-time data is used to provide real-time testing and monitoring of the patient's technique for using an inhaler and an augmented reality training aid that informs the patients training.

WO 2017/210311 A1 relates to systems and methods for data analytics-mediated interactive training e.g., for training of dental personnel in the use of automated syringe fill systems. An interactive training system is disclosed, including a personal digital appliance programmably arranged for training of an individual with audiovisual training content viewable by the individual on the personal digital appliance, wherein the audiovisual training content is integrated with corresponding success-contingent progressionary testing on the personal digital appliance of the individual's learning of the training content viewed by the individual on the personal digital appliance, as a requisite for advancement in the training. The testing includes input by the individual of responses to the personal digital appliance, as training data of the individual, and the personal digital appliance is programmably arranged for input of performance data by the individual in subsequent contemporaneous performance of trained action by the individual. The personal digital appliance inputted performance data may be used by the appliance to generate an assessment of proficiency of the individual and/or to generate other feedback to the individual in the trained action, and/or the inputted performance data may be downloadable from the personal digital appliance to generate and assessment of proficiency of the individual and/or to generate other feedback to the individual in the trained action.

US 5,791,907 A relates to an interactive medical training device including a computer system having a display, wherein the computer system is programmed to provide education and training in medical procedures, including laparoscopic surgical procedures. This aspect is achieved by configuring the system to display, on a portion of the display, a video window. The video window displays a prerecorded video segment illustrating a portion of a laparoscopic surgical procedure. The system requests a user to input information relating to a next step in the surgical procedure, which advantageously keeps the user engaged in the training session. This "next step" information may include, for example, selecting an appropriate medical instrument or selecting a location for operating. The system then receives and interprets the user input and informs the user as to whether the input is correct. Preferable, if the input is correct the system will display a prerecorded video segment illustrating the next step of the surgical procedure.

EP 3581921 A1 relates to a method for evaluating a suitability of lighting conditions for detecting an analyte in a sample using a camera of a mobile device and a detection method for detecting an analyte in a sample by using a camera of a mobile device. In case the lighting conditions are not suitable, the mobile device may, e.g., be adapted to abort and/or to prevent detecting the analyte in the sample.

US 2015/233898 A1 relates to measuring physical and biochemical parameters with mobile devices. Moving the mobile phone or shielding the test strip module by displaying a message on the screen is suggested in case of a high ambient light effect.

US 2013/330831 A1 discloses a system for water and food safety testing using a test strip and generating and analyzing a digital images of the test strip. The system can also capture user specific data such as the number of tests performed, aggregate test results, and the number of errors associated with that user's activity.

Despite the advantages achieved by the known methods and devices several technical challenges remain. Specifically, reduction of the number of failed measurements for the user, e.g. in order to avoid frustration for the user and to reduce consumption of disposables, in particular of test strips, that do not lead to meaningful measurements results, reduction of time to achieve meaningful measurement results, reliability and/or accuracy of the measurements need to be enhanced and ensured. Further, a major challenge still relies in the fact that user-specific preferences and user-specific handling of measurement devices and measurement processes differ to a wide extent. This challenge is mainly due to the fact that a wide variety of users is intended to apply the algorithms and measurement setups, having differing physical or mental capabilities and preferences.

### Problem to be solved

It is therefore desirable to provide methods and devices which address the above-mentioned technical challenges of analytical measurements using mobile devices such as consumer-electronics mobile devices, specifically multipurpose mobile devices which are not dedicated to analytical measurements such as smart phones or tablet computers. Specifically, methods and devices shall be proposed which take into account user capabilities and which, thus, automatically may adapt measurement modes to improve user handling, by still using standard mobile devices without the necessity of manually adapting device settings.

### Summary

This problem is addressed by the methods, devices, computer programs, computer-readable storage media, and kits with the features of the independent claims. Advantageous embodiments which might be realized in an isolated fashion or in any arbitrary combinations are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect, the present invention relates to:
An adjustment method for adjusting a setup for an analytical method of determining a concentration of an analyte in a bodily fluid based on a color formation reaction in an optical test strip, the analytical method comprising using a mobile device having a camera, a processor and a memory. The adjustment method comprises the following steps which specifically may be performed in the given order. It shall be noted, however, that a different order is also possible. Further, it is also possible to perform one or more of the method steps once or repeatedly. Further, it is possible to perform two or more of the method steps simultaneously or in a timely overlapping fashion. The method may comprise further method steps which are not listed. The adjustment method comprises:
a) carrying out a plurality of analyte measurement attempts with the mobile device set in a standard measuring mode, wherein each of the plurality of analyte measurement-attempts comprises
   i) capturing, by using the camera, one or more images of at least a part of an optical test strip having a test field before and/or after sample application;
   ii)checking, by the processor, for the fulfillment of one or more measurement rejection criteria stored in the memory, and if one or more measurement rejection criteria are fulfilled rejecting, by the processor, the measurement attempt and logging the one or more rejection events in the memory
b) analyzing, by the processor, one or more of the logged rejection events in an error analysis and based on the result of the error analysis adjusting the setup by placing the device in
   a reduced measuring mode wherein one or more of the measurement rejection criteria are deactivated, and/or
   an enhanced measuring mode wherein during a measurement attempt corrective feedback is provided to the user.

The term "analytical method of determining the concentration of an analyte in a bodily fluid", also simply referred to as an "analytical method" or an "analytical measurement" or an "analyte measurement" as used herein is a broad term and is to be given its ordinary and customary meaning to a person of ordinary skill in the art and is not to be limited to a special or customized meaning. The term specifically may refer, without limitation, to a quantitative and/or qualitative determination of at least one analyte in an arbitrary sample or aliquot of bodily fluid. For example, the bodily fluid may comprise one or more of blood, interstitial fluid, urine, saliva or other types of body fluids. The result of the determining of the concentration, as an example, may be a concentration of the analyte and/or the presence or absence of the analyte to be determined. In particular, the analyte may be glucose. Specifically, as an example, the analytical measurement may be a blood glucose measurement, thus the result of the analytical measurement may for example be a blood glucose concentration. In particular, an analytical measurement result value may be determined by the analytical measurement. Consequently, the term "analytical measurement result value", also referred to as an "analyte concentration value" or "analyte measurement result value", as used herein, is a broad term and is to be given its ordinary and customary meaning to a person of ordinary skill in the art and is not to be limited to a special or customized meaning. The term specifically may refer, without limitation, to a numerical indication of an analyte concentration in a sample.

The at least one analyte, as an example, may be or may comprise one or more specific chemical compounds and/or other parameters. As an example, one or more analytes may be determined which take part in metabolism, such as blood glucose. Additionally or alternatively, other types of analytes or parameters may be determined, e.g. a pH value. The at least one sample, specifically, may be or may comprise at least one bodily fluid, such as blood, interstitial fluid, urine, saliva or the like. Additionally or alternatively, however, other types of samples may be used, such as water.

The term "sample" as used herein is a broad term and is to be given its ordinary and customary meaning to a person of ordinary skill in the art and is not to be limited to a special or customized meaning. The term specifically may refer, without limitation, to an arbitrary amount of fluid for use in an analytical measurement. In particular, the sample may be a sample of bodily fluid and may be or may comprise at least 2 microliter (µl) of bodily fluid, in one embodiment at least 5 microliter (µl) of bodily fluid, such as of one or more of blood, interstitial fluid, urine, saliva and other body fluids. Specifically, the sample of bodily may comprise at least a minimum amount of bodily fluid necessary for performing an analytical measurement, specifically a minimum amount of bodily fluid for representatively determining the analyte concentration in the bodily fluid.

The analytical measurement (also referred to as "analyte measurement" herein), specifically, may be an analytical measurement including a change of at least one optical property of an optical test strip, which change may be measured or determined visually by using the camera. Specifically, the analytical measurement may be or may comprise a color formation reaction in the presence of the at least one analyte to be determined. The term "color formation reaction" as used herein is a broad term and is to be given its ordinary and customary meaning to a person of ordinary skill in the art and is not to be limited to a special or customized meaning. The term specifically may refer, without limitation, to a chemical, biological or physical reaction during which a color, specifically a reflectance, of at least one element involved in the reaction, changes with the progress of the reaction.

The term "measurement attempt" or "analyte measurement attempt" as used herein is a broad term and is to be given its ordinary and customary meaning to a person of ordinary skill in the art and is not to be limited to a special or customized meaning. The term specifically may refer, without limitation, to at least part of an analytical measurement. Specifically, the term may refer to parts of an analytical measurement based on which no analytical measurement result value will be provided to the user.

The term "setup for an analytical method of determining a concentration of an analyte in a bodily fluid" as used herein is a broad term and is to be given its ordinary and customary meaning to a person of ordinary skill in the art and is not to be limited to a special or customized meaning. The term specifically may refer, without limitation, to a configuration of a system or a configuration of a device for performing the analytical method. Specifically, the term may refer to at least one software configuration for running at least one application on the device, such as at least one software for performing the analytical measurement. Exemplary the setup may refer to, as an example, one or more of a standard measuring mode, a reduced measuring mode and an enhanced measuring mode. Generally, the term setup may thus refer to the way of performing the analytical method, determined by the software configuration. In one exemplary setup, a standard measuring mode is used. The setup may be particularly be adjusted or changed from the standard measuring mode to a reduced measuring mode or to an enhanced measuring mode.

Consequently, the term "adjustment method of adjusting a setup for an analytical method of determining a concentration of an analyte in a bodily fluid", also simply referred to as an "adjustment method", as used herein also is a broad term and is to be given its ordinary and customary meaning to a person of ordinary skill in the art and is not to be limited to a special or customized meaning. The term specifically may refer, without limitation, to a method of one or more of changing or adjusting the setup as defined above. Specifically, in the adjustment method a measurement rejection criteria may be activated or deactivated or a corrective feedback (mechanism or function) may be activated. Further exemplary embodiments will be given below.

The term "optical test strip" as used herein is a broad term and is to be given its ordinary and customary meaning to a person of ordinary skill in the art and is not to be limited to a special or customized meaning. The term specifically may refer, without limitation, to an arbitrary element or device comprising at least one strip-shaped carrier, with the at least one test field applied thereto or integrated therein, the element being configured for performing a color-change detection reaction. The optical test strip may also be referred to as a test strip or a test element. The optical test strip may particularly have a test field containing at least one test chemical, such as at least one reagent element, for detecting at least one analyte. The optical test strip, as an example, may comprise at least one substrate, such as at least one carrier, with the at least one test field applied thereto or integrated therein. In particular, the optical test strip may further comprise at least one white area, such as a white field, specifically in a proximity to the test field, for example enclosing or surrounding the test field. The white area may be a separate field independently arranged on the substrate or carrier. However, additionally or alternatively, the substrate or carrier itself may be or may comprise the white area. These test strips are generally widely in use and available. One test strip may carry a single test field or a plurality of test fields having identical or different test chemicals comprised therein.

As further used herein, the term "test field" is a broad term and is to be given its ordinary and customary meaning to a person of ordinary skill in the art and is not to be limited to a special or customized meaning. The term specifically may refer, without limitation, to a coherent amount of the test chemical, such as to a field, e.g. a field of round, polygonal or rectangular shape, having one or more layers of material, with at least one layer of the test field having the test chemical comprised therein.

The analytical method, as outlined above, comprises using a mobile device having a camera. The term "mobile device" as used herein is a broad term and is to be given its ordinary and customary meaning to a person of ordinary skill in the art and is not to be limited to a special or customized meaning. The term specifically may refer, without limitation, to a mobile electronics device, more specifically to a mobile communication device such as a cell phone or smartphone. Additionally or alternatively, as will be outlined in further detail below, the mobile device may also refer to a tablet computer or another type of portable computer having at least one camera.

The mobile device specifically, as outlined above, may comprise at least one processor. The term "processor" as generally used in this description is a broad term and is to be given its ordinary and customary meaning to a person of ordinary skill in the art and is not to be limited to a special or customized meaning. The term specifically may refer, without limitation, to an arbitrary logic circuitry configured for performing basic operations of a computer or system, and/or, generally, to a device which is configured for performing calculations or logic operations. In particular, the processor may be configured for processing basic instructions that drive the computer or system. As an example, the processor may comprise at least one arithmetic logic unit (ALU), at least one floating-point unit (FPU), such as a math coprocessor or a numeric coprocessor, a plurality of registers, specifically registers configured for supplying operands to the ALU and storing results of operations, and a memory, such as an L1 and L2 cache memory. In particular, the processor may be a multi-core processor. Specifically, the processor may be or may comprise a central processing unit (CPU). Additionally or alternatively, the processor may be or may comprise a microprocessor, thus specifically the processor's elements may be contained in one single integrated circuitry (IC) chip. Additionally or alternatively, the processor may be or may comprise one or more application-specific integrated circuits (ASICs) and/or one or more field-programmable gate arrays (FPGAs) or the like. The processor specifically may be configured, such as by software programming, for performing one or more steps of the adjustment method.

Specifically, the processor may be configured, such as by software programming, for performing one or more of the capturing, by using the camera, one or more images of at least a part of an optical test strip having a test field before and/or after a sample application, the checking for the fulfillment of one or more measurement rejection criteria stored in the memory, and if one or more measurement rejection criteria are fulfilled (based on an analysis of the at least one captured image checking), the rejecting the measurement attempt (based on the captured at least one image) (without providing an analytical measurement result value to a user), the logging the one or more rejection events in the memory, and the analyzing of one or more of the logged rejection events (from one or more measurement attempts in the past) in an error analysis and based on the error analysis (automatically) the adjusting the setup by placing the device in (instead of the standard measuring mode) a reduced measuring mode and/or an enhanced measuring mode.

The term "memory" as used herein is a broad term and is to be given its ordinary and customary meaning to a person of ordinary skill in the art and is not to be limited to a special or customized meaning. The term specifically may refer, without limitation, to any type of memory capable of storing data and retrieving that data for transfer to one or more other components of the device, such as the processor. The memory may comprise one or more of a Flash memory, SRAM, ROM, DRAM, RAM, EPROM and dynamic storage. The memory may be coupled to the processor and may be configured to receive and store one or more device parameters comprising, e.g., measurement rejection criteria, activation or deactivation of measurement rejection criteria, user input data from the touchscreen, user input from buttons or switches, time, date, sensor readings, device operating status, device messages to the user, user templates or predetermined fluid delivery patterns. The device parameters may be stored as a discrete data set at a particular point in time, a multitude of sequential discrete data sets separated by a period of time, or what is effectively termed "real-time" or continuous recording of the device parameters as fast as the system will allow. Other methods of recording device parameters such as initiating a recording based upon a trigger event are readily apparent and well known to those of skill in the art.

The term "camera" as used herein is a broad term and is to be given its ordinary and customary meaning to a person of ordinary skill in the art and is not to be limited to a special or customized meaning. The term specifically may refer, without limitation, to a device having at least one imaging element configured for recording or capturing spatially resolved one-dimensional, two-dimensional or even three-dimensional optical data or information. As an example, the camera may comprise at least one camera chip, such as at least one CCD chip and/or at least one CMOS chip configured for recording images. As used herein, without limitation, the term "image" specifically may relate to data recorded by using a camera, such as a plurality of electronic readings from the imaging device, such as the pixels of the camera chip.

The camera, besides the at least one camera chip or imaging chip, may comprise further elements, such as one or more optical elements, e.g. one or more lenses. As an example, the camera may be a fix-focus camera, having at least one lens which is fixedly adjusted with respect to the camera. Alternatively, however, the camera may also comprise one or more variable lenses which may be adjusted, automatically or manually. The invention specifically shall be applicable to cameras as usually used in mobile applications such as notebook computers, tablets or, specifically, cell phones such as smart phones. Thus, specifically, the camera may be part of a mobile device which, besides the at least one camera, comprises one or more data processing devices such as one or more data processors. Other cameras, however, are feasible.

The camera specifically may be a color camera. Thus, such as for each pixel, color information may be provided or generated, such as color values for three colors R, G, B. a larger number of color values is also feasible, such as four color values for each pixel, for example R, G, G, B. Color cameras are generally known to the skilled person. Thus, as an example, the camera chip may consist of a plurality of three or more different color sensors each, such as color recording pixels like one pixel for red (R), one pixel for green (G) and one pixel for blue (B). For each of the pixels, such as for R, G, B, values may be recorded by the pixels, such as digital values in the range of 0 to 255, depending on the intensity of the respective color. Instead of using color triples such as R, G, B, as an example, quadruples may be used, such as R, G, G, B. The color sensitivities of the pixels may be generated by color filters or by appropriate intrinsic sensitivities of the sensor elements used in the camera pixels. These techniques are generally known to the skilled person.

The term "capturing one or more images" as used herein (also referred to as "capturing at least one image" herein) is a broad term and is to be given its ordinary and customary meaning to a person of ordinary skill in the art and is not to be limited to a special or customized meaning. The term specifically may refer, without limitation, to one or more of imaging, image recording, image acquisition, image capturing. The term "capturing one or more images" may comprise capturing a single image and/or a plurality of images such as a sequence of images. For example, the capturing may comprise recording continuously a sequence of images such as a video or a movie. The capturing of the one or more images may be initiated by user action or may automatically be initiated, e.g. once the presence of the at least one object within a field of view and/or within a predetermined sector of the field of view of the camera is automatically detected. These automatic image acquisition techniques are known e.g. in the field of automatic barcode readers, such as from automatic barcode reading apps. The capturing of the one or more images may take place, as an example, by acquiring a stream or "life stream" of images with the camera, wherein one or more of the images, automatically or by user interaction such as pushing a button, are stored and used as the one or more images. The image acquisition may be supported by the processor of the mobile device, and the storing of the images may take place in the memory of the mobile device.

The at least one image of the at least one part of the optical test strip specifically comprises an image of at least a part of the test field. Further, the image may comprise an image of other parts of the optical test strip, such as a white reference part of the test strip. As another example, the image may comprise at least a part of a reference card. A color reference card may thus also be visible in the image and may be used for evaluation purposes, such as a white field. An optional reference field may thus be part of the test element or, as an example, may be part of a reference card or a reference element. The reference card or the reference element having the at least one optional reference field, such as at least one color reference field, may be placed in the field of view of the camera during capturing the image in step a) i).

The capturing of the one or more images may comprise capturing one or more images with before and/or after having the sample of the bodily fluid applied to the test strip. For example, the capturing of the one or more images may comprise, capturing at least one image without having the sample of the body fluid applied to the test strip and capturing at least one image with the sample applied to the test strip. The first image specifically may be used for calibration or comparative or quality purposes and may also be referred to as a "blank image" or "dry image". The sample application generally may take place, as an example, directly or indirectly, e.g. via at least one capillary element. The at least one image captured after sample application may typically also be referred to as the "wet image", even though the sample may have dried when the image is actually captured. As an example, at least one drop of sample, e.g. at least 2 to 5 µl of bodily fluid, may be applied to the test field by the user. For example, the sample may be dropped and/or spread onto the test field by the user. Various application techniques may be possible, such as, for example, applying the sample to the test field from a backside of the test field and capturing first and second images from the front side. The wet image typically may be taken after having waited for at least a predetermined waiting time, such as after five seconds or more, in order to allow for the detection reaction to take place. Thus, as an example, the method may comprise, between taking the at least one optional dry image and the at least one wet image, waiting for at least a predetermined minimum amount of time. This predetermined minimum amount of time specifically may be sufficient for a detection reaction to take place in the test strip. As an example, the minimum amount of waiting time may be at least 5 s.

The device may be operated in different modes including a standard measuring mode and at least one of a reduced measuring mode or an enhanced measuring mode. The term "standard measuring mode" as used herein is a broad term and is to be given its ordinary and customary meaning to a person of ordinary skill in the art and is not to be limited to a special or customized meaning. The term specifically may refer, without limitation, to a configuration of a device for performing the analytical method. Specifically, the term may refer to a setup specific for ensuring obtaining a valid measurement result. Such setup may however also include, if conditions are detected that may jeopardize obtaining a valid measurement result, rejecting the measuring attempt. In particular, this may include rejecting the measuring attempt without providing an analytical measurement result value to a user. It may further include aborting the measurement and requiring the measurement program to be started anew by the user. Generally, the term standard measuring mode may thus refer to the way of performing the analytical method including the setting or activation of one or more measurement rejection criteria and the deactivation of one or more corrective feedback functions.

Consequently, the term "placing the device in a standard measuring mode" or "setting the device in a standard measuring mode", as used herein also is a broad term and is to be given its ordinary and customary meaning to a person of ordinary skill in the art and is not to be limited to a special or customized meaning. The term specifically may refer, without limitation, to one or more of changing or adjusting the measurement or device setup as defined above. Specifically, the term may refer to changing or adjusting at least one parameter having an influence on the measurement including measurement rejection without providing an analytical measurement result value to the user. Exemplary embodiments will be given below.

The term "measurement rejection criteria" as used herein is a broad term and is to be given its ordinary and customary meaning to a person of ordinary skill in the art and is not to be limited to a special or customized meaning. The term specifically may refer, without limitation, to a characterization of measurement conditions in which case the measurement attempt is rejected in order to avoid obtaining invalid or at least doubtful measurement results. Thus, as an example, the fulfillment of measurement rejection criteria may be checked based on an analysis of the at least one captured image in step ii). In another embodiment the fulfillment of measurement rejection criteria may be checked based on an analysis of sensor data obtained from one or more sensors of the mobile device, e.g. during capturing the one or more images.

In particular, measurement rejection criteria may refer to a condition of inadmissibility of determining an analytical measurement result value. The measurement rejection criteria, as an example, may thus also be considered an item of inadmissibility information which can be Boolean or digital information, such as indicating "fulfilled" or "not fulfilled".

The measurement rejection criteria may specifically determine boundary conditions when the one or more captured images are determined as being inadmissible for the purpose of determining an analytical measurement result value.

As a simple example, one or more parameters determined by an analysis of the captured one or more images or one or more sensor data obtained from one or more sensors of the mobile device may be compared with one or more comparative values, reference values or standard values, wherein the comparison may result in a binary result such as "fulfilled" or "not fulfilled". The at least one comparative and/or reference value may comprise at least one threshold value, such as a minimum homogeneity in the region of interest (ROI). In one embodiment, comparative and/or reference values may comprise a maximum tilt angle of the mobile device e.g. vs the test strip or other comparative and/or reference values relating to the positioning of the test strip (or the test field) to the mobile device when capturing the one or more images. The comparative values, reference values and/or standard values may be derived, as an example, from experiments or from boundary conditions determined e.g. by a precision to be achieved in the analytical measurement.

As another example, a measurement rejection criteria may define an exposure threshold that is stored in the memory, the exceedance of which leading to rejecting the measurement attempt based on the captured at least one image. If, based on an analysis of the at least one captured image it is thus determined during a measurement attempt that the light conditions under which the at least one image was taken are unsuitable for determining a measurement result value, e.g. due to overexposure, the measurement attempt based on the captured at least one image is rejected. Rejection of the measurement attempt thus prevents providing an analytical measurement result value to the user.

The term "checking for the fulfillment of one or more measurement rejection criteria" as used herein is a broad term and is to be given its ordinary and customary meaning to a person of ordinary skill in the art and is not to be limited to a special or customized meaning. The term specifically may refer, without limitation, to analyzing whether one or measurement rejection criteria, which may be stored in the memory are fulfilled. A measurement rejection criteria may, e.g., be considered to be fulfilled, if an image analysis result value (based on an analysis of the one or more captured images) or a sensor data analysis result value (based on an analysis of the obtained sensor data) is obtained that falls below a predefined acceptable threshold and/or that exceeds a predefined acceptable threshold.

If a measurement rejection criteria is fulfilled in b), the measurement attempt based on the captured at least one image will be rejected. The term "rejecting the measurement attempt" as used herein is a broad term and is to be given its ordinary and customary meaning to a person of ordinary skill in the art and is not to be limited to a special or customized meaning.

The term specifically may refer, without limitation, to an instance of not providing a measurement result value to the user based on the captured one or more images. For example, the rejection of the measurement attempt may refer to a rejection of the measurement attempt based on the captured one or more images and requiring capturing one or more second images of at least a part of the optical test strip having a test field before and/or after a sample application to base a determination of an analytical measurement result value on. Specifically, the term may refer to disregarding the one or more captured images for the determination of an analytical measurement result value.

In case of rejection of the measurement attempt a note may be shown to the user on the display informing about an error or about the need to capture one or more new images. Step a) of the method may thus comprise if one or more measurement rejection criteria are fulfilled displaying an (error) message on a display of the device. In another embodiment, in case of rejection of the measurement attempt the program of the analyte measurement may need to be manually restarted by the user or continuation of the program may need to be manually confirmed by the user before undertaking a new measurement attempt.

The term "logging the one or more rejection events in the memory" as used herein is a broad term and is to be given its ordinary and customary meaning to a person of ordinary skill in the art and is not to be limited to a special or customized meaning. The term specifically may refer, without limitation, to writing the occurrence of the one or more rejection events including date and time of occurrence into the memory. Further details may also be logged, such as specific error analysis values and accompanying conditions. The measurement rejection criteria may be stored in the memory according to the first-in first out principle.

A plurality of measurement attempts thus may be conducted and measurement rejection events, if occurring, may be logged in the memory. For example, the term of a "plurality of measurement attempts" specifically may refer, without limitation, to at least two, at least 3, at least 5, at least 10, or at least 25 measurements attempts. The plurality of analyte measurement attempts may specifically be conducted by one and the same user.

The term "analyzing one or more of the logged rejection events in an error analysis" as used herein is a broad term and is to be given its ordinary and customary meaning to a person of ordinary skill in the art and is not to be limited to a special or customized meaning. The term specifically may refer, without limitation, to a process for deriving statistics and/or information from logged rejection events. Thus, specifically, the error analysis may, e.g., comprise using statistical analysis to determine a rejection event logged most often, or logged more often than a predefined threshold as a result of the error analysis. Other statistical analysis, however, may also be conducted. The error analysis may further determine whether one or more predefined rejection events were logged at all. The analysis of the one or more logged rejection events in the error analysis may be limited to an analysis of a predefined number N_{A} of most recent measurement attempts or a predefined number N_{R} of most recent logged rejection events or a predefined timespan.

Based on the result of the error analysis the setup of the device is adjusted by placing the device in a reduced measuring mode and/or an enhanced measuring mode. For example, the placing of the device in a reduced measuring mode with a specific one or more measurement rejection criteria being deactivated may be made dependent on a predefined rejection event occurring at all, a rejection event occurring more often than a predefined threshold, or a rejection event occurring most often over all rejection events or over a certain timespan, or the rejection event having occurred most recently.

The placing of the device in a reduced measuring mode and/or an enhanced measuring mode may thus be dependent on the result the error analysis and optionally occur automatically upon a predefined result being obtained from the error analysis. The term of a "reduced measuring mode and an enhanced measuring mode" refers to a combination of the reduced measuring mode and the enhanced measuring mode, wherein one or more of the measurement rejection criteria are deactivated and wherein during a measurement attempt corrective feedback is provided to the user.

The term "reduced measuring mode" may specifically refer, without limitation, to a configuration of a device for performing the analytical method. Specifically, the term may refer to a setup specific for training certain aspects of the analytical measurement by the user. In the reduced measuring mode at least one measurement rejection criteria which is activated in the standard mode is deactivated. The reduced measuring mode may thus correspond to the standard measuring mode, wherein however, one or more measurement rejection criteria are deactivated that had been activated in the standard measuring mode. Deactivation may, e.g., mean that fulfillment of the one or more deactivated measurement rejection criteria is not checked for in the reduced measuring mode, or, if it is checked for, fulfillment will not lead to rejecting the measurement attempt. Generally, the term "reduced measuring mode" may thus refer to a training mode.

Consequently, the term "placing the device in a reduced measuring mode" may specifically refer, without limitation, to one or more of changing or adjusting the measurement or device setup as defined above. Specifically, the term may refer to deactivating at least one measurement rejection criteria. A training result may thus be provided to the user despite the fact that certain requirements which would be necessary in the standard measuring mode are not fulfilled or not checked for. As an example, the user may train positioning the test strip to the of the camera for capturing the at least one image without having to worry about homogeneity of the region of interest.

The term "enhanced measuring mode" may specifically refer, without limitation, to a configuration of a device for performing the analytical method. Specifically, the term may refer to, as an example, a setup specific for supporting aspects of the analytical measurement by the user. In the enhanced measuring mode corrective feedback is provided to the user (e.g., to avoid one or more errors that previously occurred when the device was set in the standard measuring mode).

The enhanced measuring mode may thus correspond to the standard measuring mode, wherein however corrective feedback is provided additionally in order to avoid a rejection event to occur. Corrective feedback may, e.g., refer to an alert, a message, an invitation, or a summoning, or a request to the user. The aim is to thereby avoid one or more rejection events occurring. Generally, the term "enhanced measuring mode" may thus refer to the way of performing a analytical measurement method with additional support to the user. Specifically, the corrective feedback may be provided to the user via the display of the device.

Consequently, the term "placing the device in an enhanced measuring mode"may specifically refer, without limitation, to one or more of changing or adjusting the measurement or device setup as defined above. Specifically, the term may refer to activating at least one corrective feedback function that had not been active in the standard measuring mode. A successful analytical measurement may thus be facilitated. In one embodiment, the corrective feedback provided to the user shall avoid the occurrence of rejection events and support capturing one or more images of at least a part of an optical test strip having a test field which will not be disregarded in the determination of an analytical measurement result value. As an example, before capturing one or more images in the enhanced measuring mode, a message may be displayed to the user on the device to pay attention to the correct position of the test strip to the mobile device or the camera for capturing the one or more images of the of at least a part of an optical test strip having a test field.

The adjustment method may comprise checking in step a) ii), by the processor, for the fulfillment of one or more measurement rejection criteria stored in the memory based on an analysis of the one or more images captured in step a) i). From the image analysis one or more image analysis result values (also called parameters) may be determined. The image analysis result values may be compared with comparative values, reference values or standard values stored in the memory function. Such comparative values, reference values or standard values may thus function as thresholds values. An image analysis result value obtained that falls below a predefined acceptable threshold and/or that exceeds a predefined acceptable threshold may thus indicate fulfillment of a measurement rejection criteria.

The adjustment method of the present invention may comprise
in step a) i) obtaining sensor data by using at least one sensor of the mobile device and
in step a) ii) checking for the fulfillment of one or more measurement rejection criteria stored in the memory based on one or more of the obtained sensor data.

Sensor data may be obtained by using at least one sensor of the users' mobile phone, specifically at least one of: an inclination sensor; a light sensor; a motion sensor; an acceleration sensor; a gyroscopic sensor; a magnetic sensor, a Hall sensor; a GPS sensor; a pressure sensor, a barometer; a temperature sensor; a biometric sensor, specifically one or more of a fingerprint sensor and/or an iris scan sensor. In one embodiment, the term sensor data may refer to data received other from the color sensors of the camera.

One or more of the sensor data obtained from one or more sensors (or further processed data derived from the sensor data) of the mobile device may be compared with one or more comparative values, reference values or standard values stored in the memory. Such comparative values, reference values or standard values may thus function as thresholds values. Sensor data that fall below a corresponding predefined acceptable threshold and/or that exceeds a corresponding predefined acceptable threshold may thus indicate fulfillment of a measurement rejection criteria.

The measurement rejection criteria of the present invention may specifically comprise at least one of
insufficient image sharpness,
insufficient ROI saturation
insufficient ROI size,
insufficient ROI homogeneity,
insufficient steadiness, and
insufficient orientation

The sharpness check may ensure that the recorded image is not blurred too much. For a blurred image, the region of interest (ROI) may not be properly distinguishable.

The term "region of interest" as used herein is a broad term and is to be given its ordinary and customary meaning to a person of ordinary skill in the art and is not to be limited to a special or customized meaning. The term may specifically refer, without limitation, to a section or a segment or a partition of an object, wherein the section, segment or partition is identified for a particular purpose. Thus, the region of interest may e.g. be a delimited surface area of the object. Alternatively, the region of interest may refer to a subset of data of an image, wherein the subset represents the section, segment or partition of the object. As an example, the region of interest may comprise certain information or information may be deducible from it. The region of interest (ROI) may, e.g., define those pixels in the image which belong to the corresponding test field or at least a predefined part of the corresponding test field. Other regions of interests (on the test strip) may also be determined, e.g. for obtaining reference values. A region of interest may thus also comprise at least one element of the group consisting of a white field and a black field.

Image sharpness may specifically be determined by the distance of a pixel level between a lower (x, e.g. 10 or 15%) to a higher (y, e.g. 85 or 90%) of its final value (also called x-y rise distance). In one embodiment, the gradient of the black-to-white transition of the strip's black field to the adjacent white field may be determined. Insufficient image sharpness thus may be a sharpness below or a above a predefined sharpness threshold value, e.g. an x-y distance above a predefined corresponding threshold.

The saturation check tests if the minimum pixel value in a ROI is below a certain limit and/or if the maximum pixel value in the ROI is above a certain limit. Insufficient ROI saturation thus may refer to a situation wherein the minimum pixel value in the ROI is below a first predefined saturation threshold value and/or wherein the maximum pixel value in the ROI is above a second predefined saturation threshold value, e.g. above 250 (for R, G and/or B).

The homogeneity check tests whether a ROI is homogenous. An inhomogenous region of interest may, e.g., be an indication of a contaminated test field or a light shadow or an insufficient sample size applied to the test field. ROI homogeneity thus may specifically be determined by comparing mean or median (or one or more other statistical parameters) of intensity values of subregions of the region of interest. A difference between the one or more statistical values being above a predefined homogeneity threshold value may be indicative for an insufficient ROI homogeneity.

The ROI size test checks if the pixel count for a determined region of interest is below a certain limit and/or is above a certain limit. Insufficient ROI size may thus refer to a situation wherein the pixel count for a determined region of interest is below a first predefined ROI size threshold value and/or wherein the pixel count for a determined region of interest is above a second predefined ROI size threshold value, This may ensure an adequate distance between the test strip and the mobile device or the camera. It may also ensure a representative image analysis. The ROI size analysis may be conducted before and/or after outlier removal using the same or different threshold values.

The steadiness check tests if the mobile device was kept steady during capturing the one or more images. Insufficient steadiness may refer to a situation wherein the motion sensor data indicate motion above a predefined motion threshold value, e.g., when the mobile phone is still in motion when capturing the one or more images.

The orientation check is for ensuring a correct orientation of the camera to the test strip. Insufficient orientation may comprise a maximum tilt angle of the mobile device as an orientation threshold angle (e.g. vs the test strip or other comparative and/or reference values relating to the positioning of the test strip (or the test field) to the mobile device when capturing the one or more images) being exceeded.

The adjustment method may further comprise
a) iii) determining and providing, by the processor, an analytical measurement result value if the measurement attempt was not rejected in step a) ii).

The adjustment method may comprise
a) iii) determining and providing, by the processor, an analytical measurement result value by using the one or more images captured in step a) i) if the measurement attempt was not rejected in step a) ii).

The analytical measurement result value, as an example, may be a numerical value indicator of a result of the analytical measurement, such as indicative of the concentration of at least one analyte in the sample, such as a blood glucose concentration. For determining the analytical measurement result value from the one or more captured images, as an example, a predetermined or determinable relationship between information derived from one or more images, such as color information or color change information, and the at least one analytical measurement result value may be used. This predetermined or determinable relationship, as an example, may be stored in the memory of the mobile device and/or in the processor of the mobile device. The processor, as an example, may be configured by software programming to derive at least one item of information (also called parameter) from the one or more captured images, such as at least one color coordinate, and to apply the predetermined or determinable relationship to the at least one item of information The correlation, as an example a transformation function, a transformation table or a lookup table, may be determined e.g. empirically and may, as an example, be stored in at least one memory of the mobile device, e.g. by the software, specifically by the app downloaded from an app store or the like. As an example for deriving the at least one item of information, the processor may be programmed in order to recognize, preferably automatically, e.g. by pattern recognition and/or other algorithms, the test field or the at least one part of the test field in the images. Thereof, the processor may be programmed for determining the at least one item of information, such as one or more color coordinates. The respective at least one item of information derived from the image before sample application (blank image) may be used for normalizing, such as by dividing the at least one item of information derived from the image after sample application (wet image) by the at least one item of information derived from the corresponding blank image or by subtracting the at least one item of information derived from the wet image from the at least one item of information derived from the blank image or vice a versa. Other ways of normalizing are feasible. The correlation, as an example a transformation function, a transformation table or a lookup table, may be determined e.g. empirically and may, as an example, be stored in at least one data storage device of the mobile device, e.g. by the software, specifically by the app downloaded from an app store or the like.

The method may further comprise the step of displaying the analytical measurement result value, such as on a display of the mobile device. Therein, one or more numerical values indicating the analytical measurement result value may be indicated, e.g. on a display. Additionally or alternatively, range indication regarding the analytical measurement result value may be indicated, such as by indicating whether the analytical measurement result value is within or without one or more predefined ranges. As an example, a displaying of ranges such as high range, target range or in low range may be performed, without necessarily indicating the numerical value of the analytical measurement result value. Thus, the display not necessarily needs to be the display of a numerical value. Other means of displaying the analytical measurement result value are feasible, wherein the display may be one or more of a visual display, an audible display or a haptic display. Additionally or alternatively, the method may comprise storing the analytical measurement result value in at least one data storage device of the mobile device. Again additionally and alternatively, the method may further comprise transmitting the analytical measurement result value via at least one interface and/or via at least one data transmission network, such as to another computer, e.g. for further evaluation.

The method may comprise
in step a) i) capturing, by using the camera, at least one image of at least a part of an optical test strip having a test field before and at least one image of at least a part of an optical test strip after sample application,
in step a) ii) checking, by the processor, for the fulfillment of one or more measurement rejection criteria stored in the memory based on an analysis of the at least two captured images, and if one or more measurement rejection criteria are fulfilled rejecting, by the processor, the measurement attempt and logging the one or more rejection events in the memory, and
in step a) iii) determining, by the processor, an analytical measurement result value by using at least one of the at least two images and providing the analytical measurement result value to the user if the measurement attempt was not rejected based on the analysis of the at least two images.

The method may also comprises a step of asking the user to confirm that a sample of bodily fluid was applied to the test field and receipt of this confirmation may be taken as start of the reaction. The at least one image captured after sample application should then be captured within a predefined time span after the start of the reaction. Thereby, measurement performance may be improved by excluding too short or overly long reaction times of the sample with the reagent system as too short or overly long reaction times may lead to wrong analytical measurement results.

The time point of capturing the at least one image captured after sample application in comparison to the time point of the start of the reaction may be considered the reaction time. A timestamp indicating the date and time when an image has been captured may be associated (and optionally saved, at least temporarily) with each image captured, respectively. This allows for an easy determination or the reaction time based on the timestamp of the captured image and the start of the reaction time, which may be determined as described above.

As an alternative, a plurality of images of at least a part of an optical test strip may be captured in step a) i) in order to determine a wetting-induced change of optical properties occurring upon sample application. The occurrence of the wetting induced change may be taken as start of the reaction. The analytical measurement value may then be determined based at least on one of the plurality images that was taken in a predefined time span (e.g., 3 to 8 seconds or 5 to 8 seconds) from an image being taken that is indicative of the start of the reaction.

A measurement rejection criteria may thus be an insufficient reaction time. A reaction time is insufficient if it is below or above predefined lower or upper reaction time thresholds, respectively.

Another measurement rejection criteria may be an insufficient illumination consistency.

The illumination consistency test checks if the absolute difference between pixel values of corresponding regions of interests (or one or more other statistical parameters derived from pixel values of corresponding regions of interest) in two images is below a certain limit and/or is above a certain limit. Insufficient illumination consistency thus may refer to a situation wherein the absolute difference between pixel values of corresponding regions of interests (or one or more other statistical parameters derived from pixel values of corresponding regions of interest) in two images is below a first predefined illumination consistency threshold value or above a second predefined illumination consistency threshold value, respectively.

For example, step a) ii) may thus comprise checking, by the processor, for the fulfillment of insufficient illumination consistency stored in the memory based on an analysis of the at least two captured images, and if one or more measurement rejection criteria is fulfilled rejecting, by the processor, the measurement attempt and logging the rejection event in the memory.

The method of the present invention may further comprise
in step a) i) capturing, by using the camera, at least two images of at least a part of an optical test strip having a test field after sample application,
in step a) ii) checking, by the processor, for the fulfillment of insufficient illumination consistency stored in the memory based on an analysis of the at least two captured images of at least a part of an optical test strip having a test field after sample application, and if one or more measurement rejection criteria are fulfilled rejecting, by the processor, the measurement attempt and logging the one or more rejection events in the memory.

A corresponding procedure may be followed for comparing two images captured before sample application.

The method may comprise in step b) analyzing only the one or more rejection events logged from measurement attempts conducted when the mobile device was set in the standard measuring mode.

This prevents bias to the error analysis as the user might be experimenting when the device is set into a reduced and/or enhanced measuring mode.

The method of the present invention may also relate to a method, wherein the analyzed one or more logged rejection events are the one or more logged rejection events from one or more of
- N_{A} most recent measurement attempts,
- N_{R} most recent logged rejection events,
- a timespan T.

Thereby, it may be ensured that the user profits from a most up to date analysis when using the device in the reduced or enhanced measuring mode. The user can thus, e.g., practice to avoid his most recent mistakes in the reduced measuring mode or be reminded not to repeat them in the enhanced measuring mode.

The number N_{A} of most recent measurement attempts may, e.g. be 2, 3, 5, 10 or 25. The number N_{R} of most recent logged rejection events may, e.g. be 2, 3, 5, 10 or 25. The timespan T may, e.g., be the most recent 3, 5, 10, 25 or 30 days.

The term "most recent" as generally used in this description is a broad term and is to be given its ordinary and customary meaning to a person of ordinary skill in the art and is not to be limited to a special or customized meaning. The term specifically may refer, without limitation, to one or more incidences having occurred closest in time to a reference time point. In particular, the reference time point may be the time when the error analysis is started.

The method of the present invention may relate to a method, wherein the error analysis comprises determining the rejection event logged most often or logged most recently.

Alternatively or additionally, one or more other statistical parameters may be determined in the error analysis. As a further example, the error analysis may comprise determining one or more rejection events logged more often than a predefined threshold.

Based on the result of the error analysis, in other words based on what has been determined in the error analysis, the setup of the device is adjusted by placing the device in a reduced measuring mode and/or an enhanced measuring mode. Upon the result of the error analysis meeting predefined criteria, the setup may, e.g., be adjusted automatically. In the reduced measuring mode, specifically one or more of the measurement rejection criteria may be deactivated dependent on the result of the error analysis

The method of the present invention may thus relate to a method, wherein one or more, but not all measurement rejection criteria of the standard measuring mode are deactivated.

The one or more deactivated measurement rejection criteria may, in particular, be different from the measurement rejection event logged most often or most recently as determined in the error analysis. In one example, the one or more deactivated measurement rejection criteria may, in particular, be different from the one or more measurement rejection events logged more often than a predefined threshold in the error analysis.

The adjustment method may comprise
c) carrying out at least one analyte measurement attempt with the mobile device set in the reduced measuring, the analyte measurement attempt in the reduced measuring mode comprising
i) capturing, by using the camera, one or more images of at least a part of an optical test strip having a test field before and/or after sample application or of a dummy test strip,
ii) checking, by the processor, for the fulfillment of one or more measurement rejection criteria stored in the memory that are not deactivated, and optionally
iii) if one or more measurement rejection criteria which are not deactivated are fulfilled rejecting, by the processor, the measurement attempt.

What has been described in relation to steps a) i), ii) and iii) may analogously be applicable with respect to steps c) i), ii) and iii), respectively except that in the reduced measuring mode one or more of the measurement rejection criteria are deactivated which are activated in the standard measuring mode. As an example, the method may comprise in step c) ii) checking, by the processor, for the fulfillment of one or more measurement rejection criteria stored in the memory that are not activated based on an analysis of the one or more images captured in step c) i), or based on sensor data.

As a further example, the method may comprise in step c) iii) determining and providing, by the processor, an analytical measurement result value by using the one or more images captured in step c) i).

In case the checking of step c) ii) results in the finding that one or more measurement rejection criteria stored in the memory that are not deactivated are fulfilled, a note may be shown to the user on the display informing about an error.

As another example of the method of the present invention, the method may comprise, in case the checking of step c) ii) results in the finding that no measurement rejection criteria stored in the memory that is not deactivated is fulfilled, displaying a positive message on the display of the device to the user. Thereby, the user who might have experienced a particular measurement rejection event, may receive positive reinforcement when then successfully conducting a method step that has caused him difficulties before.

As the reduced measuring mode may be considered a training mode, in step c) i) a dummy test strip may be used to train capturing images therefrom which will not be disregarded in the analytical method of determining a concentration of an analyte in a bodily fluid due to causing a rejection event based on image analysis. A dummy test strip may, specifically, relate to a test strip imprint on a test strip package or a previously used test strip, i.e. a test strip with a dried sample. For training, the user thus does not have to waste fresh test strips.

The adjustment method of the present invention also relates to a method comprising
c) carrying out at least one analyte measurement attempt with the mobile device set in the enhanced measuring wherein the corrective feedback is provided to the user before capturing, by using the camera, one or more images of at least part of an optical test strip having a test field before and/or after a sample application or of a dummy test strip.

The adjustment method may further comprise
i) capturing, by using the camera, one or more images of at least a part of an optical test strip having a test field before and/or after sample application or of a dummy test strip,
ii) checking by the processor, for the fulfillment of one or more measurement rejection criteria stored in the memory, and optionally
iii) determining and providing, by the processor, an analytical measurement result value if the measurement attempt was not rejected in step c) ii).

What has been described in relation to steps a) i), ii) and iii) may also be applicable analogously with respect to steps c) i), ii) and iii), except that in the enhanced measuring mode one or more corrective feedback functions are activated which are not activated in the standard measuring mode.

As an example, the method may comprise in step c) ii) checking, by the processor, for the fulfillment of one or more measurement rejection criteria stored in the memory based on an analysis of the one or more images captured in step c) i), or based on sensor data.

As a further example, the method may comprise in step c) iii) determining and providing, by the processor, an analytical measurement result value by using the one or more images captured in step c) i).

A measurement attempt may specifically start with detecting, by the processor, a user interaction with the device that is related to conducting an analytical measurement. For example, start of a measurement attempt may be detected by the user opening the App of the present invention on a device, or by pressing a button or tapping on the touchpad of the device (after opening the App when prompted). A measurement attempt may end with its rejection. Providing corrective feedback during a measurement attempt may thus help the user to proceed successfully through an analytical measurement method. Providing the feedback during the measurement attempt also aims at avoiding rejection events shortly before they might actually happen instead of, e.g. providing feedback or a report on rejection events to the user when he might be less interested (because he is not actually trying to do a measurement at that point in time).

The corrective feedback relates to the result of the error analysis. As an example, if the error analysis comprises determining the rejection event logged most often or logged most recently or determining one or more rejection events logged more often than a predefined threshold, the corrective feedback may be a message alerting the user to avoid the corresponding one or more rejection events to happen. The corrective feedback provided during a measurement attempt may further be restricted to up to three messages or only one message during the measurement attempt. This may ensure that the user will not be overwhelmed with information.

The method, in one or more embodiments disclosed, may be fully or partially computer-implemented. Thus, in a further aspect, a computer program is proposed comprising instructions which, when the program is executed by a mobile device having a memory and a camera, specifically by a processor of the mobile phone, cause the mobile device to carry out the method as described herein, more specifically at least steps a) ii) and b), and optionally one or more of a) iii), c) ii) and c) iii) as described herein. Further, also other steps, such as a) i) and/or c) i) of the method may, at least partially, be computer-implemented or at least computer-supported.

The computer program specifically may be designed as an application, e.g. as an App. The App, as an example, may be downloaded onto the mobile device from a download server.

The computer program may further comprise instructions that, when the program is executed by the mobile device, further prompt a used to perform or confirm the performance of certain steps.

In a further aspect, a computer-readable storage medium is disclosed, specifically a non-transitory storage medium, comprising instructions which, when executed by a mobile device having a camera, a processor and a memory, cause the mobile device, cause the mobile device to carry out the method according to the present invention, such as according to any one of the embodiments disclosed above and/or according to any one of the embodiments disclosed in further detail below, more specifically, at least steps a) ii) and b), and optionally any one or more of steps a) iii), c) ii) and c) iii) as described herein. Further, also other steps, such as a) i) and/or c) i) of the method may, at least partially, be computer-implemented or at least computer-supported.

The computer-readable storage medium may further comprise instructions which, when executed by the mobile device, prompt a user to perform or confirm the performance of one or more steps.

As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

The computer program may also be embodied as a computer program product. As used herein, a computer program product may refer to the program as a tradable product. The product may generally exist in an arbitrary format, such as in a paper format, or on a computer-readable data carrier and/or on a computer-readable storage medium. Specifically, the computer program product may be distributed over a data network.

In a further aspect, a mobile device for performing an analytical measurement is disclosed. For definitions and options of the mobile device, reference may be made to the description of the method given above or as further outlined below. The mobile device comprises at least one camera, at least one display, at least one processor, at least one memory. The mobile device is configured for performing at least steps a) ii) and b), and optionally any one or more of steps a) i), a) iii), c) i), c) ii) and c) iii) of the method according to the present invention, such as according to any one of the embodiments disclosed above and/or according to any one of the embodiments described in further detail below. Thus, the processor of the mobile device may be software-configured for performing and/or controlling the execution of at least steps a) ii) and b) of the method, wherein also steps a) i), a) iii) and c) i), c)ii) and c) iii) may at least partially be controlled and/or supported by the processor.

As outlined above, the mobile device may comprise at least one processor being programmed for controlling at least one of steps a) ii) and b), and optionally, at least partially, one or more of a) i), a) iii) and c) i), c)ii) and c) iii) of the method of the present invention. For definitions and options regarding the design of the processor, reference may be made to the description given above.

In a further aspect, the present invention relates to a kit comprising
- at least one of the device, computer program instructions, and computer-readable storage medium as described herein before, and
- at least one of a test strip and a dummy test strip.

The term "kit" as used herein is a broad term and is to be given its ordinary and customary meaning to a person of ordinary skill in the art and is not to be limited to a special or customized meaning. The term specifically may refer, without limitation, to an assembly of a plurality of components, wherein the components each may function and may be handled independently from each other, wherein the components of the kit may interact to perform a common function.

The kit may further comprise at least one reference card, the reference card having at least one reference color field, e.g. a white field and/or a black field.

Generally, the invention may greatly help users in conducting analytical measurements and in the handling of their mobile device for conducting analytical measurements. It may also improve measurement performance of analytical measurements in the end if the user is better trained to use his device.

Summarizing the following embodiments are defined in the claims:
Embodiment 1:
   An adjustment method for adjusting a setup for an analytical method of determining a concentration of an analyte in a bodily fluid based on a color formation reaction in an optical test strip, the analytical method comprising using a mobile device having a camera, a processor and a memory, wherein the adjustment method comprises:
   a) carrying out a plurality of analyte measurement attempts with the mobile device set in a standard measuring mode, wherein each of the plurality of analyte measurement -attempts comprises
      i) capturing, by using the camera, one or more images of at least a part of an optical test strip having a test field before and/or after sample application;
      ii)checking, by the processor, for the fulfillment of one or more measurement rejection criteria stored in the memory, and if one or more measurement rejection criteria are fulfilled rejecting, by the processor, the measurement attempt and logging the one or more rejection events in the memory,
   b) analyzing, by the processor, one or more of the logged rejection events in an error analysis and based on the result of the error analysis adjusting the setup by placing the device in
      a reduced measuring mode wherein one or more of the measurement rejection criteria are deactivated, and/or
      an enhanced measuring mode wherein during a measurement attempt corrective feedback is provided to the user.
Embodiment 2:
   The adjustment method according to the preceding embodiment, wherein the analyte is glucose.
Embodiment 3:
   The adjustment method according to any of the preceding embodiments, wherein the sample is a sample of bodily fluid, in particular blood.
Embodiment 4:
   The adjustment method according to any of the preceding embodiments, wherein step a) comprises if one or more measurement rejection criteria are fulfilled displaying an error message on a display of the device.
Embodiment 5:
   The adjustment method according to any of the preceding embodiments, wherein step a) ii) comprises checking, by the processor, for the fulfillment of one or more measurement rejection criteria stored in the memory based on an analysis of the one or more images captured in step a) i).
Embodiment 6:
   The adjustment method according to any of the preceding embodiments comprising in step a) i) obtaining sensor data by using at least one sensor the mobile device and in step a) ii) checking for the fulfillment of one or more measurement rejection criteria stored in the memory based on one or more of the obtained sensor data.
Embodiment 7:
   The adjustment method according to any of the preceding embodiments, wherein the one or more measurement rejection criteria comprise at least one of
   insufficient image sharpness,
   insufficient ROI saturation
   insufficient ROI size,
   insufficient ROI homogeneity,
   insufficient steadiness, and
   insufficient orientation.
Embodiment 8:
   The adjustment method according to any of the preceding embodiments comprising
   a) iii) determining and providing, by the processor, an analytical measurement result value if the measurement attempt was not rejected in step a) ii).
Embodiment 9:
   The adjustment method according to any of the preceding embodiments comprising
   a) iii) determining and providing, by the processor, an analytical measurement result value by using the one or more images captured in step a) i) if the measurement attempt was not rejected in step a) ii).
Embodiment 10:
   The adjustment method according to any of the preceding embodiments comprising displaying the analytical measurement result value on a display of the mobile device.
Embodiment 11:
   The adjustment method according to any of the preceding embodiments, wherein step a) i) comprises capturing, by using the camera, at least one image of at least a part of an optical test strip having a test field before and at least one image of at least a part of an optical test strip after sample application,
   step a) ii) comprises checking, by the processor, for the fulfillment of one or more measurement rejection criteria stored in the memory based on an analysis of the at least two captured images, and if one or more measurement rejection criteria are fulfilled rejecting, by the processor, the measurement attempt and logging the one or more rejection events in the memory, and
   step a) iii) comprises determining, by the processor, an analytical measurement result value by using at least one of the at least two images and providing the analytical measurement result value to the user if the measurement attempt was not rejected based on the analysis of the at least two images.
Embodiment 12:
   The adjustment method according to any of the preceding embodiments wherein the measurement rejection criteria is an insufficient reaction time and/or an insufficient illumination consistency.
Embodiment 13:
   The adjustment method according to any of the preceding embodiments, wherein step b) comprises analyzing only the one or more rejection events logged from measurement attempts conducted when the mobile device was set in the standard measuring mode.
Embodiment 14:
   The adjustment method according to any of the preceding embodiments, wherein the analyzed one or more logged rejection events are the one or more logged rejection events from one or more of
   - N_{A} most recent measurement attempts,
   - N_{R} most recent logged rejection events,
   - a timespan T.
Embodiment 15:
   The adjustment method according to any of the preceding embodiments, wherein the error analysis comprises determining the rejection event logged most often or logged most recently.
Embodiment 16:
   The adjustment method according to any of the preceding embodiments, wherein one or more, but not all measurement rejection criteria of the standard measuring mode are deactivated.
Embodiment 17:
   The adjustment method according to any of the preceding embodiments, wherein the one or more deactivated measurement rejection criteria are different from the measurement rejection event logged most often or most recently as determined in the error analysis.
Embodiment 18:
   The adjustment method according to any of the preceding embodiments comprising
   c) carrying out at least one analyte measurement attempt with the mobile device set in the reduced measuring, the analyte measurement attempt in the reduced measuring mode comprising
   i) capturing, by using the camera, one or more images of at least a part of an optical test strip having a test field before and/or after sample application or of a dummy test strip,
   ii) checking, by the processor, for the fulfillment of one or more measurement rejection criteria stored in the memory that are not deactivated, and optionally
   iii) if one or more measurement rejection criteria which are not deactivated are fulfilled rejecting, by the processor, the measurement attempt.
Embodiment 19:
   The adjustment method according to any of the preceding embodiments comprising
   c) carrying out at least one analyte measurement attempt with the mobile device set in the enhanced measuring wherein the corrective feedback is provided to the user before capturing, by using the camera, one or more images of at least a part of an optical test strip having a test field before and/or after sample application or of a dummy test strip.
Embodiment 20:
   The adjustment method according to any of the preceding embodiments, wherein rejection events occurred while the device is set in the reduced and/or enhanced measuring mode are not logged in the memory.
Embodiment 21:
   A mobile device having at least at least a camera, a processor and a memory, the mobile device being configured for:
   performing at least steps a) and b), and optionally step c) of the method according to any of the preceding embodiments.
Embodiment 22:
   A computer program comprising instructions which, when the program is executed by a mobile device having a camera, a memory and a processor cause the mobile device to carry out at least method steps a) ii) and b), and optionally any one or more of steps a) i), a) iii), c) i), c) ii) and c) iii) of any of the preceding method embodiments.
Embodiment 23:
   A computer-readable storage medium comprising instructions which, when executed by a mobile device having a camera, a processor and a memory, cause the mobile device to carry out at least method steps a) ii) and b), and optionally any one or more of steps a) i), a) iii), c) i), c) ii) and c) iii) of any of the preceding method embodiments.
Embodiment 24:
   A kit comprising
   - at least one of the device, computer program instructions, and computer-readable storage medium of any of the respective preceding embodiments, and
   - at least one of a test strip and a dummy test strip.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows an embodiment of a kit and of a mobile device.
- Figures 2 and 3: show flow charts of embodiments of the adjustment method; and
- Figure 4: shows a flow chart of an embodiment of a measurement attempt.

### Detailed description of the embodiments

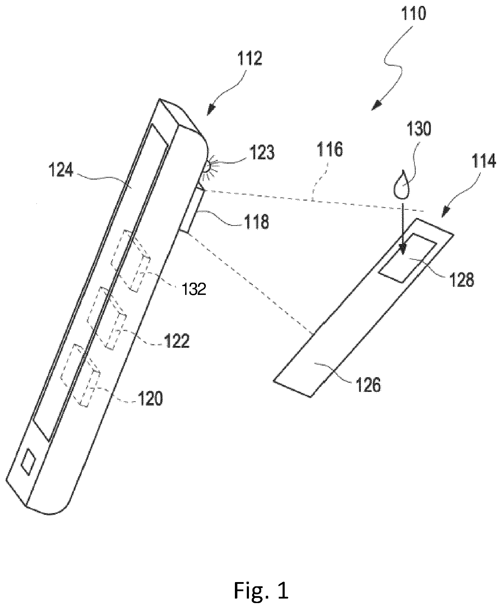

In Figure 1, an exemplary embodiment of a kit 110 is shown in a perspective view. The kit 110 comprises a mobile device 112 such as a smart phone and, further, at least one optical test strip 114, in this setup placed in a field of view 116 of a camera 118 of the mobile device 112.

The mobile device 112 may comprise, besides the at least one camera 118, at least one processor 120, at least one memory 122, and at least one sensor 132. The mobile device 112 may further comprise at least one display 124, such as for displaying a life image taken by the camera 118 and/or for displaying information to a user. The mobile device 112 may further comprise at least one illumination source 123 such as an LED or the like.

The optical test strip 114 may comprise at least one substrate 126 such as a flexible, strip-shaped substrate. The optical test strip 114 further comprises at least one test field 128 applied to the substrate, the test field 128 comprising at least one test chemical for performing a detection reaction with at least one analyte comprised by a sample 130. The sample 130 may directly or indirectly be applied to the test field 128, such as by applying a droplet of a bodily fluid to the test field 128 and/or to a capillary element for conducting the sample 130 to the test field 128.

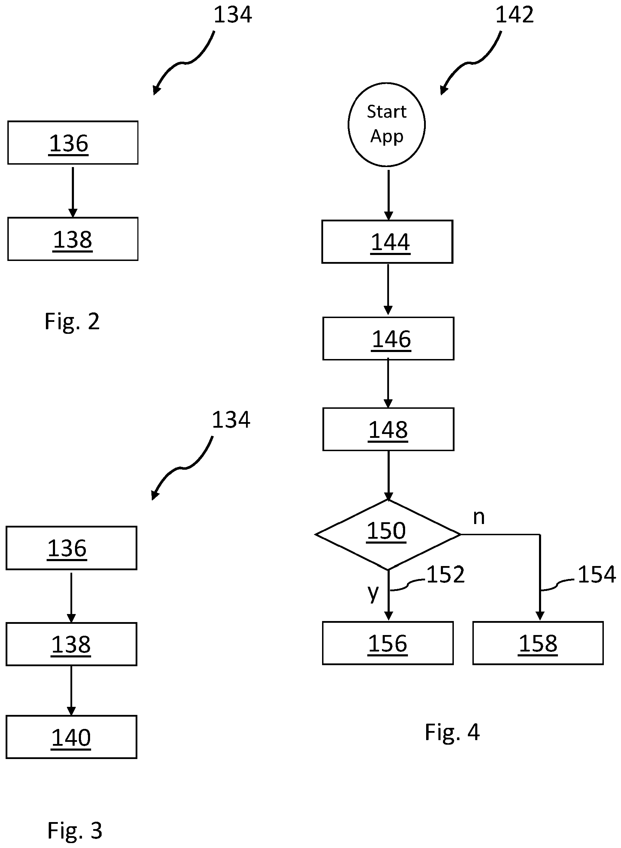

The mobile device 112 is configured, by appropriate programming of the processor 120, for performing and/or supporting the method according to the present invention which will be described with reference to an exemplary embodiment shown in a flow chart in Figure 2.

A flow chart of an exemplary embodiment of an adjustment method 134 is shown in Figure 2. The adjustment method 134 comprises the following steps, which may specifically be performed in the given order. Still, a different order may also be possible. It may be possible to perform two or more of the method steps fully or partially simultaneously. It may further be possible to perform one, more than one or even all of the method steps once or repeatedly. The adjustment method 134 comprises:
a) (denoted with reference number 136) carrying out, a plurality of analyte measurement attempts with the mobile device (114) set in a standard measuring mode, wherein each of the plurality of analyte measurement-attempts comprises
   i) capturing, by using the camera (118), one or more images of at least a part of an optical test strip (114) having a test field (128) before and/or after sample (130) application;
   ii) checking, by the processor (120), for the fulfillment of one or more measurement rejection criteria stored in the memory (122), and if one or more measurement rejection criteria are fulfilled rejecting, by the processor (120), the measurement attempt and logging the one or more rejection events in the memory (122),
b) (denoted with reference number 138) analyzing, by the processor (120), one or more of the logged rejection events in an error analysis and based on the result of the error analysis adjusting the setup by placing the device (112) in
   a reduced measuring mode wherein one or more of the measurement rejection criteria are deactivated, and/or
   an enhanced measuring mode wherein during a measurement attempt corrective feedback is provided to the user.

The adjustment method 134 may comprise additional method steps that are not listed in Fig. 2. As exemplarily illustrated by Figure 3, the method 134 may comprise an additional step
c) (denoted with reference number 140) carrying out at least one analyte measurement attempt with the mobile device set in the reduced measuring mode and/or set in the advanced measuring mode.

A flow chart of an exemplary embodiment of a measurement attempt 142 is shown in Figure 4. Specifically, measurement attempt 142 may start, for example, with starting an App on the mobile device, e.g. the smartphone. An optical test strip (114) having a test field (128) may be provided (denoted with reference number 144). The measurement attempt further comprises.
i) capturing (denoted with reference number 146), by using the camera (118), one or more images of at least a part of an optical test strip (114) having a test field (128) before and/or after sample (130) application; and
ii) checking (denoted with reference number 148) for the fulfillment of one or more measurement rejection criteria stored in the memory (122).

The branching point 150 may indicate a condition query, such as deciding between a first branch 152 and a second branch 154. As an example, the first branch 152 may indicate fulfillment ("y") of one or more measurement rejection criteria. Thus, the first branch 152 leads to the step (denoted with reference number 156) of rejecting the measurement attempt and logging the one or more rejection events in the memory (122). The second branch 154 may indicate non-fulfillment ("n") of one or more measurement rejection criteria and, thus, may lead to iii) (denoted with reference number 158) determining and providing, by the processor, an analytical measurement result value.

### List of reference numbers

- 110: kit
- 112: mobile device
- 114: optical test strip
- 116: field of view
- 118: camera
- 120: processor
- 122: memory
- 123: illumination source
- 124: display
- 126: substrate
- 128: test field
- 130: sample
- 132: sensor
- 134: adjustment method
- 136: step a)
- 138: step b)
- 140: step c)
- 142: measurement attempt
- 144: providing an optical test strip
- 146: step a) i)
- 148: checking for the fulfillment of one or more measurement rejection criteria stored in the memory
- 150: branching point
- 152: first branch
- 154: second branch
- 156: rejecting the measurement attempt and logging the one or more rejection events in the memory
- 158: step a) iii)

## Claims

1. An adjustment method (134) for adjusting a setup for an analytical method of determining a concentration of an analyte in a bodily fluid based on a color formation reaction in an optical test strip, the analytical method comprising using a mobile device (112) having a camera (118), a processor (120) and a memory (122), wherein the adjustment method comprises:
a) carrying out a plurality of analyte measurement attempts (142) with the mobile device (112) set in a standard measuring mode, wherein each of the plurality of analyte measurement -attempts (142) comprises
i) capturing, by using the camera (118), one or more images of at least a part of an optical test strip having a test field (128) before and/or after sample application;
ii) checking, by the processor (120), for the fulfillment of one or more measurement rejection criteria stored in the memory (122), and if one or more measurement rejection criteria are fulfilled rejecting, by the processor (120), the measurement attempt (142) and logging the one or more rejection events in the memory (122),
b) analyzing, by the processor (120), one or more of the logged rejection events in an error analysis and based on the result of the error analysis adjusting the setup by placing the device in
a reduced measuring mode wherein one or more of the measurement rejection criteria are deactivated, and/or
an enhanced measuring mode wherein during a measurement attempt (142) corrective feedback is provided to the user.

2. The adjustment method according to any of the preceding claims, wherein step a) ii) comprises checking, by the processor (120), for the fulfillment of one or more measurement rejection criteria stored in the memory (122) based on an analysis of the one or more images captured in step a) i).

3. The adjustment method according to any of the preceding claims comprising in step a) i) obtaining sensor data by using at least one sensor (132) of the mobile device (112) and in step a) ii) checking for the fulfillment of one or more measurement rejection criteria stored in the memory (122) based on one or more of the obtained sensor data.

4. The adjustment method according to any of the preceding claims, wherein the one or more measurement rejection criteria comprise at least one of
insufficient image sharpness,
insufficient ROI saturation
insufficient ROI size,
insufficient ROI homogeneity,
insufficient steadiness, and
insufficient orientation.

5. The adjustment method according to any of the preceding claims comprising
a) iii) determining and providing, by the processor (120), an analytical measurement result value by using the one or more images captured in step a) i) if the measurement attempt (142) was not rejected in step a) ii).

6. The adjustment method according to any of the preceding claims, wherein
step a) i) comprises capturing, by using the camera (118), at least one image of at least a part of an optical test strip having a test field (128) before and at least one image of at least a part of an optical test strip (114) after sample application,
step a) ii) comprises checking, by the processor (120), for the fulfillment of one or more measurement rejection criteria stored in the memory (122) based on an analysis of the at least two captured images, and if one or more measurement rejection criteria are fulfilled rejecting, by the processor (120), the measurement attempt (142) and logging the one or more rejection events in the memory (122), and
step a) iii) comprises determining, by the processor (120), an analytical measurement result value by using at least one of the at least two images and providing the analytical measurement result value to the user if the measurement attempt (142) was not rejected based on the analysis of the at least two images.

7. The adjustment method according to any of the preceding claims, wherein step b) comprises analyzing only the one or more rejection events logged from measurement attempts (142) conducted when the mobile device (112) was set in the standard measuring mode.

8. The adjustment method according to any of the preceding claims, wherein the analyzed one or more logged rejection events are the one or more logged rejection events from one or more of
- N_{A} most recent measurement attempts (142),
- N_{R} most recent logged rejection events,
- a timespan T.

9. The adjustment method according to any of the preceding claims, wherein the error analysis comprises determining the rejection event logged most often or logged most recently.

10. The adjustment method according to any of the preceding claims, wherein the one or more deactivated measurement rejection criteria are different from the measurement rejection event logged most often or most recently as determined in the error analysis.

11. The adjustment method according to any of the preceding claims comprising
c) carrying out at least one analyte measurement attempt with the mobile device set in the reduced measuring, the analyte measurement attempt in the reduced measuring mode comprising
i) capturing, by using the camera, one or more images of at least a part of an optical test strip having a test field before and/or after sample application or of a dummy test strip,
ii) checking, by the processor, for the fulfillment of one or more measurement rejection criteria stored in the memory that are not deactivated, and optionally
iii) if one or more measurement rejection criteria which are not deactivated are fulfilled rejecting, by the processor, the measurement attempt.

12. The adjustment method according to any of the preceding claims comprising
c) carrying out at least one analyte measurement attempt (142) with the mobile device (112) set in the enhanced measuring wherein the corrective feedback is provided to the user before capturing, by using the camera (118), one or more images of at least a part of an optical test strip having a test field (128) before and/or after sample application or of a dummy test strip.

13. A mobile device (112) having at least at least a camera (118), a processor (120) and a memory (122), the mobile device (112) being configured for:
performing at least steps a) ii) and b), and optionally step c) of the method according to any one of the preceding claims.

14. A computer program comprising instructions which, when the program is executed by a mobile device- (112) having a camera (118), a memory (122) and a processor (120) cause the mobile device (112) to carry out at least method steps a) ii) and b), and optionally any one or more of steps a) i), a) iii), c) i), c) ii) and c) iii) of any of the preceding method claims.

15. A computer-readable storage medium comprising instructions which, when executed by a mobile device (112) having a camera (118), a processor (120) and a memory (122), cause the mobile device (112) to carry out at least method steps a) ii) and b), and optionally any one or more of steps a) i), a) iii), c) i), c) ii) and c) iii) of any of the preceding method claims.

16. A kit (110) comprising
- at least one of the device, computer program instructions, and computer-readable storage medium of any of the respective preceding claims, and
- at least one of a test strip and a dummy test strip.

## Patentansprüche

1. Anpassungsverfahren (134) zum Einstellen einer Einrichtung für ein analytisches Verfahren zum Bestimmen einer Konzentration eines Analyten in einer Körperflüssigkeit, basierend auf einer Farbbildungsreaktion in einem optischen Teststreifen, wobei das analytische Verfahren das Verwenden einer mobilen Vorrichtung (112) mit einer Kamera (118), einem Prozessor (120) und einem Speicher (122) umfasst, wobei das Anpassungsverfahren Folgendes umfasst:
a) Durchführen einer Vielzahl von Analytmessversuchen (142) mit der mobilen Vorrichtung (112), die mit einem Standardmessmodus eingerichtet ist, wobei jeder der Vielzahl von Analytmessversuchen (142) Folgendes umfasst
i) Aufnehmen eines Bildes oder mehrerer Bilder von mindestens einem Teil eines optischen Teststreifens mit einem Testfeld (128) vor und/oder nach der Probenaufbringung unter Verwendung der Kamera (118);
ii) Prüfen der Erfüllung eines oder mehrerer Messungszurückweisungskriterien, die in dem Speicher (122) gespeichert sind, mittels des Prozessors (120) und, wenn ein oder mehrere Messungszurückweisungskriterien erfüllt sind, Zurückweisen des Messversuches (142) und Registrieren des einen oder der mehreren Zurückweisungsereignisse in dem Speicher (122) mittels des Prozessors (120),
b) Analysieren eines oder mehrerer der registrierten Zurückweisungsereignisse in einer Fehleranalyse mittels des Prozessors (120) und, basierend auf dem Ergebnis der Fehleranalyse, Einstellen der Einrichtung durch Setzen der Vorrichtung in einen eingeschränkten Messmodus, in dem ein oder mehrere Zurückweisungsereignisse deaktiviert werden, und/oder
einen erweiterten Messmodus, in dem der Benutzer während eines Messversuches (142) eine korrigierende Rückmeldung erhält.

2. Anpassungsverfahren nach einem der vorstehenden Ansprüche, wobei Schritt a) ii) das Prüfen der Erfüllung eines oder mehrerer Messungszurückweisungskriterien, die in dem Speicher (122) gespeichert sind, mittels des Prozessors (120) basierend auf einer Analyse des einen oder der mehreren Bilder, die in Schritt a) i) aufgenommen wurden, umfasst.

3. Anpassungsverfahren nach einem der vorstehenden Ansprüche, umfassend in Schritt a) i) das Erhalten von Sensordaten unter Verwendung mindestens eines Sensors (132) der mobilen Vorrichtung (112) und in Schritt a) ii) das Prüfen der Erfüllung eines oder mehrerer Messungszurückweisungskriterien, die in dem Speicher (122) gespeichert sind, basierend auf den erhaltenen Sensordaten.

4. Anpassungsverfahren nach einem der vorstehenden Ansprüche, wobei das eine oder die mehreren Messungszurückweisungskriterien mindestens eines der Folgenden umfassen
unzureichende Bildschärfe,
unzureichende ROI-Sättigung,
unzureichende ROI-Größe,
unzureichende ROI-Homogenität,
unzureichende Stabilität und
unzureichende Ausrichtung.

5. Anpassungsverfahren nach einem der vorstehenden Ansprüche, umfassend
a) iii) das Bestimmen und Bereitstellen eines Ergebniswertes der analytischen Messung unter Verwendung des einen oder der mehreren Bilder, die in Schritt a) i) aufgenommen wurden, wenn der Messversuch (142) in Schritt a) ii) nicht zurückgewiesen wurde, mittels des Prozessors (120).

6. Anpassungsverfahren nach einem der vorstehenden Ansprüche, wobei Schritt a) i) das Aufnehmen mindestens eines Bildes von mindestens einem Teil eines optischen Teststreifens mit einem Testfeld (128) vor und mindestens eines Bildes von mindestens einem Teil eines optischen Teststreifens (114) nach der Probenaufbringung unter Verwendung der Kamera (118) umfasst,
Schritt a) ii) das Prüfen der Erfüllung eines oder mehrerer Messungszurückweisungskriterien, die in dem Speicher (122) gespeichert sind, basierend auf einer Analyse der mindestens zwei aufgenommen Bilder mittels des Prozessors (120) und, wenn ein oder mehrere Messungszurückweisungskriterien erfüllt sind, Zurückweisen des Messversuches (142) und Registrieren des einen oder der mehreren Zurückweisungsereignisse in dem Speicher (122) mittels des Prozessors (120) umfasst, und
Schritt a) iii) das Bestimmen eines Ergebniswertes der analytischen Messung unter Verwendung mindestens eines der mindestens zwei Bilder und Bereitstellen des Ergebniswertes der analytischen Messung dem Benutzer, wenn der Messversuch (142), basierend auf der Analyse der mindestens zwei Bilder, nicht zurückgewiesen wurde, mittels des Prozessors (120) umfasst.

7. Anpassungsverfahren nach einem der vorstehenden Ansprüche, wobei Schritt b) das Analysieren lediglich des einen oder der mehreren Zurückweisungsereignisse, die aus den Messversuchen (142) registriert wurden, die durchgeführt wurden, als die mobile Vorrichtung (112) im Standardmessmodus eingerichtet war, umfasst.

8. Anpassungsverfahren nach einem der vorstehenden Ansprüche, wobei das eine oder die mehreren analysierten registrierten Zurückweisungsereignisse das eine oder die mehreren registrierten Zurückweisungsereignisse von einem oder mehreren der Folgenden sind
- N_{A} jüngste Messversuche (142),
- N_{R} jüngste registrierte Zurückweisungsereignisse,
- eine Zeitspanne T.

9. Anpassungsverfahren nach einem der vorstehenden Ansprüche, wobei die Fehleranalyse das Bestimmen des Zurückweisungsereignisses, das am häufigsten registriert wurde oder das zuletzt registriert wurde, umfasst.

10. Anpassungsverfahren nach einem der vorstehenden Ansprüche, wobei das eine oder die mehreren deaktivierten Messungszurückweisungskriterien von dem Messungszurückweisungsereignis, das am häufigsten oder zuletzt registriert wurde, verschieden sind, wie in der Fehleranalyse bestimmt.

11. Anpassungsverfahren nach einem der vorstehenden Ansprüche, umfassend
c) Durchführen mindestens eines Analytmessversuches mit der für eingeschränktes Messen eingerichteten mobilen Vorrichtung, wobei der Analytmessversuch im eingeschränkten Messmodus Folgendes umfasst
i) Aufnehmen eines oder mehrerer Bilder von mindestens einem Teil eines optischen Teststreifens mit einem Testfeld vor und/oder nach der Probenaufbringung oder von einem Blind-Teststreifen mit der Kamera,
ii) Prüfen der Erfüllung eines oder mehrerer Messungszurückweisungskriterien, die in dem Speicher gespeichert und nicht deaktiviert sind, mittels des Prozessors und gegebenenfalls
iii) wenn ein oder mehrere Messungszurückweisungskriterien, die nicht deaktiviert sind, erfüllt ist/sind, Zurückweisen des Messversuches mittels des Prozessors.

12. Anpassungsverfahren nach einem der vorstehenden Ansprüche, umfassend
c) Durchführen mindestens eines Analytmessversuches (142) mit der für erweitertes Messen eingerichteten mobilen Vorrichtung (112), wobei der Benutzer die korrigierende Rückmeldung vor dem Aufnehmen eines oder mehrerer Bilder von mindestens einem Teil eines optischen Teststreifens mit einem Testfeld (128) vor und/oder nach der Probenaufbringung oder von einem Blind-Teststreifen mit der Kamera (118) erhält.

13. Mobile Vorrichtung (112) mit mindestens einer Kamera (118), einem Prozessor (120) und einem Speicher (122), wobei die mobile Vorrichtung (112) für Folgendes ausgebildet ist:
Ausführen von mindestens Schritt a) ii) und b) und gegebenenfalls Schritt c) des Verfahrens nach einem der vorstehenden Ansprüche.

14. Computerprogramm, umfassend Anweisungen, die, wenn das Programm von einer mobilen Vorrichtung (112) mit einer Kamera (118), einem Speicher (122) und einem Prozessor (120) ausgeführt wird, die mobile Vorrichtung (112) veranlassen, mindestens Verfahrensschritt a) ii) und b) und gegebenenfalls einen oder mehrere von Schritt a) i), a) iii), c) i), c) ii) und c) iii) nach einem der vorstehenden Verfahrensansprüche auszuführen.

15. Computerlesbares Speichermedium, umfassend Anweisungen, die, wenn von einer mobilen Vorrichtung (112) mit einer Kamera (118), einem Prozessor (120) und einem Speicher (122) ausgeführt, die mobile Vorrichtung (112) veranlassen, mindestens Verfahrensschritt a) ii) und b) und gegebenenfalls einen oder mehrere von Schritt a) i), a) iii), c) i), c) ii) und c) iii) nach einem der vorstehenden Verfahrensansprüche auszuführen.

16. Kit (110), umfassend
- mindestens eines von der Vorrichtung, den Computerprogrammanweisungen und dem computerlesbaren Speichermedium nach einem der jeweiligen vorstehenden Ansprüche und
- mindestens eines von einem Teststreifen und einem Blind-Teststreifen.

## Revendications

1. Procédé de réglage (134) pour régler une configuration pour un procédé analytique de détermination d'une concentration d'un analyte dans un fluide corporel sur la base d'une réaction de formation de couleur dans une bandelette de test optique, le procédé analytique comprenant l'utilisation d'un dispositif mobile (112) ayant une caméra (118), un processeur (120) et une mémoire (122), dans lequel le procédé de réglage comprend :
a) la réalisation d'une pluralité de tentatives de mesure d'analyte (142) avec le dispositif mobile (112) configuré dans un mode de mesure standard, dans lequel chacune de la pluralité de tentatives de mesure d'analyte (142) comprend
i) la capture, en utilisant la caméra (118), d'une ou plusieurs images d'au moins une partie d'une bandelette de test optique ayant un champ de test (128) avant et/ou après application d'un échantillon ;
ii) la vérification, par le processeur (120), de l'accomplissement d'un ou plusieurs critères de rejet de mesure stockés dans la mémoire (122), et si un ou plusieurs critères de rejet de mesure sont accomplis, le rejet, par le processeur (120), de la tentative de mesure (142) et la journalisation du ou des événements de rejet dans la mémoire (122),
b) l'analyse, par le processeur (120), d'un ou plusieurs des événements de rejet journalisés dans une analyse d'erreurs et sur la base du résultat de l'analyse d'erreurs le réglage de la configuration en mettant le dispositif dans
un mode de mesure réduit dans lequel un ou plusieurs des critères de rejet de mesure sont désactivés, et/ou
un mode de mesure amélioré dans lequel pendant une tentative de mesure (142) une rétroaction corrective est fournie à l'utilisateur.

2. Procédé de réglage selon l'une quelconque des revendications précédentes, dans lequel l'étape a) ii) comprend la vérification, par le processeur (120), de l'accomplissement d'un ou plusieurs critères de rejet de mesure stockés dans la mémoire (122) sur la base d'une analyse de la ou des images capturées à l'étape a) i).

3. Procédé de réglage selon l'une quelconque des revendications précédentes comprenant à l'étape a) i) l'obtention de données de capteur en utilisant au moins un capteur (132) du dispositif mobile (112) et à l'étape a) ii) la vérification de l'accomplissement d'un ou plusieurs critères de rejet de mesure stockés dans la mémoire (122) sur la base d'une ou plusieurs des données de capteur obtenues.

4. Procédé de réglage selon l'une quelconque des revendications précédentes, dans lequel le ou les critères de rejet de mesure comprennent au moins l'un parmi
une netteté d'image insuffisante,
une saturation de ROI insuffisante
une taille de ROI insuffisante,
une homogénéité de ROI insuffisante,
une stabilité insuffisante, et
une orientation insuffisante.

5. Procédé de réglage selon l'une quelconque des revendications précédentes comprenant a) iii) la détermination et la fourniture, par le processeur (120), d'une valeur de résultat de mesure analytique en utilisant la ou les images capturées à l'étape a) i) si la tentative de mesure (142) n'a pas été rejetée à l'étape a) ii).

6. Procédé de réglage selon l'une quelconque des revendications précédentes, dans lequel l'étape a) i) comprend la capture, en utilisant la caméra (118), d'au moins une image d'au moins une partie d'une bandelette de test optique ayant un champ de test (128) avant et d'au moins une image d'au moins une partie d'une bandelette de test optique (114) après application d'un échantillon,
l'étape a) ii) comprend la vérification, par le processeur (120), de l'accomplissement d'un ou plusieurs critères de rejet de mesure stockés dans la mémoire (122) sur la base d'une analyse des au moins deux images capturées, et si un ou plusieurs critères de rejet de mesure sont accomplis, le rejet, par le processeur (120), de la tentative de mesure (142) et la journalisation du ou des événements de rejet dans la mémoire (122), et
l'étape a) iii) comprend la détermination, par le processeur (120), d'une valeur de résultat de mesure analytique en utilisant au moins l'une des au moins deux images et la fourniture de la valeur de résultat de mesure analytique à l'utilisateur si la tentative de mesure (142) n'a pas été rejetée sur la base des au moins deux images.

7. Procédé de réglage selon l'une quelconque des revendications précédentes, dans lequel l'étape b) comprend l'analyse uniquement du ou des événements de rejet journalisés à partir de tentatives de mesure (142) menées lorsque le dispositif mobile (112) a été configuré dans le mode de mesure standard.

8. Procédé de réglage selon l'une quelconque des revendications précédentes, dans lequel le ou les événements de rejet journalisés analysés sont le ou les événements de rejet journalisés d'un ou plusieurs parmi
- les tentatives de mesure les plus récentes N_{A} (142),
- les événements de rejet journalisés les plus récents N_{R},
- un laps de temps T.

9. Procédé de réglage selon l'une quelconque des revendications précédentes, dans lequel l'analyse d'erreurs comprend la détermination de l'événement de rejet journalisé le plus souvent ou journalisé le plus récemment.

10. Procédé de réglage selon l'une quelconque des revendications précédentes, dans lequel le ou les critères de rejet de mesure désactivés sont différents de l'événement de rejet de mesure journalisé le plus souvent ou le plus récemment comme déterminé dans l'analyse d'erreurs.

11. Procédé de réglage selon l'une quelconque des revendications précédentes comprenant
c) la réalisation d'au moins une tentative de mesure d'analyte avec le dispositif mobile configuré dans la mesure réduite, la tentative de mesure d'analyte dans le mode de mesure réduit comprenant
i) la capture, en utilisant la caméra, d'une ou plusieurs images d'au moins une partie d'une bandelette de test optique ayant un champ de test avant et/ou après application d'un échantillon ou d'une bandelette de test factice,
ii) la vérification, par le processeur, de l'accomplissement d'un ou plusieurs critères de rejet de mesure stockés dans la mémoire qui ne sont pas désactivés, et éventuellement
iii) si un ou plusieurs critères de rejet de mesure qui ne sont pas désactivés sont accomplis, le rejet, par le processeur, de la tentative de mesure.

12. Procédé de réglage selon l'une quelconque des revendications précédentes comprenant
c) la réalisation d'au moins une tentative de mesure d'analyte (142) avec le dispositif mobile (112) configuré dans la mesure améliorée dans laquelle la rétroaction corrective est fournie à l'utilisateur avant la capture, en utilisant la caméra (118), d'une ou plusieurs images d'au moins une partie d'une bandelette de test optique ayant un champ de test (128) avant et/ou après application d'un échantillon ou d'une bandelette de test factice.

13. Dispositif mobile (112) ayant au moins une caméra (118), un processeur (120) et une mémoire (122), le dispositif mobile (112) étant configuré pour :
effectuer au moins les étapes a) ii) et b), et éventuellement l'étape c) du procédé selon l'une quelconque des revendications précédentes.

14. Programme informatique comprenant des instructions qui, lorsque le programme est exécuté par un dispositif mobile (112) ayant une caméra (118), une mémoire (122) et un processeur (120) amènent le dispositif mobile (112) à réaliser au moins les étapes a) ii) et b) du procédé, et éventuellement l'une quelconque ou plusieurs des étapes a) i), a) iii), c) i), c) ii) et c) iii) de l'une quelconque des revendications précédentes du procédé.

15. Support de stockage lisible par ordinateur comprenant des instructions qui, lorsqu'elles sont exécutées par un dispositif mobile (112) ayant une caméra (118), un processeur (120) et une mémoire (122), amènent le dispositif mobile (112) à réaliser au moins les étapes a) ii) et b) du procédé, et éventuellement l'une quelconque ou plusieurs des étapes a) i), a) iii), c) i), c) ii) et c) iii) de l'une quelconque des revendications précédentes du procédé.

16. Kit (110) comprenant
- au moins l'un parmi le dispositif, les instructions de programme informatique et le support de stockage lisible par ordinateur de l'une quelconque des revendications précédentes respectives, et
- au moins l'une parmi une bandelette de test et une bandelette de test factice.
